# EUROPEAN PATENT SPECIFICATION

(11) **EP 4 218 603 B1**
(45) Date of publication and mention of the grant of the patent: **17.12.2025**
(21) Application number: 21872181.9
(22) Date of filing: 09.09.2021
(51) Int. Cl.: A61B 8/14, A61B 6/00

(54) **ULTRASONIC DIAGNOSTIC DEVICE AND METHOD FOR CONTROLLING ULTRASONIC DIAGNOSTIC DEVICE**
ULTRASCHALLDIAGNOSEVORRICHTUNG UND VERFAHREN ZUR STEUERUNG DER ULTRASCHALLDIAGNOSEVORRICHTUNG
DISPOSITIF DE DIAGNOSTIC À ULTRASONS ET PROCÉDÉ DE COMMANDE DE DISPOSITIF DE DIAGNOSTIC À ULTRASONS

(30) Priority: 28.09.2020 JP 2020162263
(43) Date of publication of application: 02.08.2023
(60) Divisional application: 25211113.3
(73) Proprietor: FUJIFILM Corporation, Tokyo 106-8620 (JP)
(72) Inventor: KOSHINO, Riko, Tokyo 106-8620 (JP)
(74) Representative: Dehns Germany Partnerschaft mbB
(86) International application number: PCT/JP2021/033135
(87) International publication number: WO 2022/065050

(56) References cited:
- JP-A- 2004 248 818
- JP-A- 2008 086 742
- JP-A- 2015 100 661
- JP-A- 2015 112 123
- JP-A- 2015 231 438
- JP-A- 2018 050 761
- US-A1- 2018 249 985
- US-A1- 2019 096 065
- US-B1- 6 396 940

## Description

### BACKGROUND OF THE INVENTION

### 1. Field of the Invention

The present invention relates to an ultrasound diagnostic apparatus for examining a breast of a subject and a control method for the ultrasound diagnostic apparatus.

### 2. Description of the Related Art

Conventionally, an examination on a lesion part or the like in a subject has been performed by using an ultrasound diagnostic apparatus. Prior to an examination using such an ultrasound diagnostic apparatus, an examination in a subject is often performed in advance by using an image diagnostic apparatus different from the ultrasound diagnostic apparatus, such as a computed tomography (CT) apparatus. In this case, a user such as a doctor often observes both an ultrasound image captured by the ultrasound diagnostic apparatus and a medical image captured by the other image diagnostic apparatus to make a diagnosis on the lesion part or the like of a subject.

As described above, in order to improve the accuracy of diagnosis using two different medical images, for example, an ultrasound diagnostic apparatus disclosed in JP2020-39877A has been developed. JP2020-39877A discloses a technique in which a two-dimensional CT cross-section image representing a cross section corresponding to an ultrasound image is selected on the basis of three-dimensional data of a subject obtained through CT imaging, and the selected CT cross-section image and the ultrasound image are displayed. US 2018/249985 A1 discloses an ultrasound diagnostic apparatus comprising an image generation unit that generates an ultrasound image including a region of interest of a breast of a subject captured in a radiation image by transmitting and receiving ultrasound beams to and from the subject by using the ultrasound probe. It further comprises an image adjustment unit that adjusts the radiation image and the ultrasound image such that the region of interest captured in the ultrasound image and the region of interest captured in the radiation image have an identical orientation on the basis of radiation image orientation information stored in a tag of the radiation image and probe orientation information of the ultrasound probe in a case where the ultrasound image is captured, and a monitor that displays the radiation image and the ultrasound image that have been adjusted by the image adjustment unit.

### SUMMARY OF THE INVENTION

Incidentally, in a case where an examination using an ultrasound diagnostic apparatus is performed on a breast of a subject, an examination called mammography is often performed before the examination using the ultrasound diagnostic apparatus.

Here, in both the examination using the ultrasound diagnostic apparatus and the examination using the CT apparatus as disclosed in JP2020-39877A, a subject is subjected to the examination while lying on an examination table or the like. Consequently, in the examination using the ultrasound diagnostic apparatus and the examination using the CT apparatus, the breasts of the subject are imaged from an identical orientation, and shapes of the breasts of the subject at the time of imaging may be identical to each other. Therefore, in a case where an examination using the CT apparatus is performed prior to the examination using the ultrasound diagnostic apparatus, it is possible to easily obtain an ultrasound image and a CT cross-section image that are easy for a user to compare.

However, in a case of capturing a radiation image of a breast of a subject by using mammography, imaging is performed in a state in which the subject is standing, and imaging is performed in a state in which the breast is compressed by a so-called compression plate and an imaging table. Therefore, a shape of the breast of the subject in the mammography and a shape of the breast of the subject in the examination using the ultrasound diagnostic apparatus are different from each other. Thus, there is a problem that it is difficult to capture a radiation image representing an identical cross section as a cross section represented by an ultrasound image, and it is difficult for a user such as a doctor to compare the ultrasound image with the radiation image.

The present invention has been made in view of such a conventional problem, and an object thereof is to provide a ultrasound diagnostic apparatus and a control method for the ultrasound diagnostic apparatus enabling a user to easily compare an ultrasound image with a radiation image and capable of improving diagnostic accuracy for a subject.

According to the present invention, there is provided an ultrasound diagnostic apparatus as claimed in claim 1.

It is preferable that the probe orientation information is position information of the ultrasound probe designated by a user or position information detected by a position sensor mounted on the ultrasound probe.

The image adjustment unit may generate the adjusted radiation image and ultrasound image by performing at least one of a rotation process or an inversion process on at least one of an entire radiation image or an entire ultrasound image.

Alternatively, the ultrasound diagnostic apparatus may further include a region-of-interest extraction unit that extracts the region of interest from each of the radiation image and the ultrasound image, and the image adjustment unit may generate the adjusted radiation image and ultrasound image by performing at least one of a rotation process or an inversion process on at least one of the region of interest extracted from the radiation image or the region of interest extracted from the ultrasound image.

The image adjustment unit may display a subject orientation mark representing an orientation of the subject on the adjusted radiation image and ultrasound image to be superimposed.

The image adjustment unit generates the adjusted radiation image and ultrasound image such that the region of interest captured in the radiation image and the region of interest captured in the ultrasound image have an identical size.

The image adjustment unit determines a ratio between sizes of the adjusted radiation image and ultrasound image on the basis of a stored inter-pixel distance of the radiation image and a stored inter-pixel distance of the ultrasound image.

It is preferable that, in a case where the radiation image is acquired in a state in which a radiation source is disposed in a direction inclined with respect to a vertical direction, the image adjustment unit performs rotational conversion on the radiation image on the basis of a rotation angle of the radiation source and then adjusts the radiation image and the ultrasound image.

The tag of the radiation image may include radiation image breast information indicating whether the breast of the subject captured in the radiation image is a left or right breast, and, in a case where the breast of the subject captured in the radiation image and the breast of the subject captured in the ultrasound image match each other, the image adjustment unit may adjust the radiation image and the ultrasound image on the basis of the radiation image breast information and information input by the user and representing whether the breast of the subject captured in the ultrasound image is a left or right breast.

The image adjustment unit may further adjust the already adjusted radiation image and ultrasound image on the basis of readjustment information input by the user.

According to the present invention, there is provided a control method as claimed in claim 9.

According to the present invention, the ultrasound diagnostic apparatus includes an image adjustment unit that adjusts the radiation image and the ultrasound image such that the region of interest captured in the ultrasound image and the region of interest captured in the radiation image have an identical orientation on the basis of radiation image orientation information stored in a tag of the radiation image and probe orientation information of the ultrasound probe in a case where the ultrasound image is captured; and a monitor that displays the radiation image and the ultrasound image that have been adjusted by the image adjustment unit. Therefore, a user can easily compare the ultrasound image with the radiation image, and can improve the diagnostic accuracy for a subject.

### BRIEF DESCRIPTION OF THE DRAWINGS

Fig. 1 is a block diagram showing a configuration of an ultrasound diagnostic apparatus according to Embodiment 1 of the present invention.
Fig. 2 is a schematic diagram of an example of a radiation image stored in a server according to Embodiment 1 of the present invention.
Fig. 3 is a schematic diagram of an ultrasound probe according to Embodiment 1 of the present invention.
Fig. 4 is a block diagram showing a configuration of a transmission/reception circuit according to Embodiment 1 of the present invention.
Fig. 5 is a block diagram showing a configuration of an image generation unit according to Embodiment 1 of the present invention.
Fig. 6 is a schematic diagram of an example of an ultrasound image according to Embodiment 1 of the present invention.
Fig. 7 is a schematic diagram of an example of a radiation image adjusted in Embodiment 1 of the present invention.
Fig. 8 is a flowchart showing an operation of the ultrasound diagnostic apparatus according to Embodiment 1 of the present invention.
Fig. 9 is a schematic diagram of an example of a radiation image and an ultrasound image displayed on a monitor according to Embodiment 1 of the present invention.
Fig. 10 is a schematic diagram of another example of a radiation image and an ultrasound image displayed on the monitor in Embodiment 1 of the present invention.
Fig. 11 is a schematic diagram of another example of the radiation image adjusted in Embodiment 1 of the present invention.
Fig. 12 is a schematic diagram of an example of a sub-window on a radiation image and a sub-window on an ultrasound image displayed on the monitor in Embodiment 1 of the present invention.
Fig. 13 is a schematic diagram of an example of a region of interest displayed in a sub-window according to Embodiment 1 of the present invention.
Fig. 14 is a block diagram showing a configuration of an ultrasound diagnostic apparatus according to Embodiment 2 of the present invention.
Fig. 15 is a block diagram showing a configuration of an ultrasound diagnostic apparatus according to Embodiment 3 of the present invention.

### DESCRIPTION OF THE PREFERRED EMBODIMENTS

Hereinafter, embodiments of the present invention will be described with reference to the accompanying drawings.

The following description of the constitutional requirements is based on a representative embodiment of the present invention, but the present invention is not limited to such an embodiment.

In the present specification, a numerical range represented by using "to" means a range including the numerical values described before and after "to" as a lower limit value and an upper limit value.

In the present specification, "identical" and "similar" include an error range generally allowed in the technical field.

### Embodiment 1

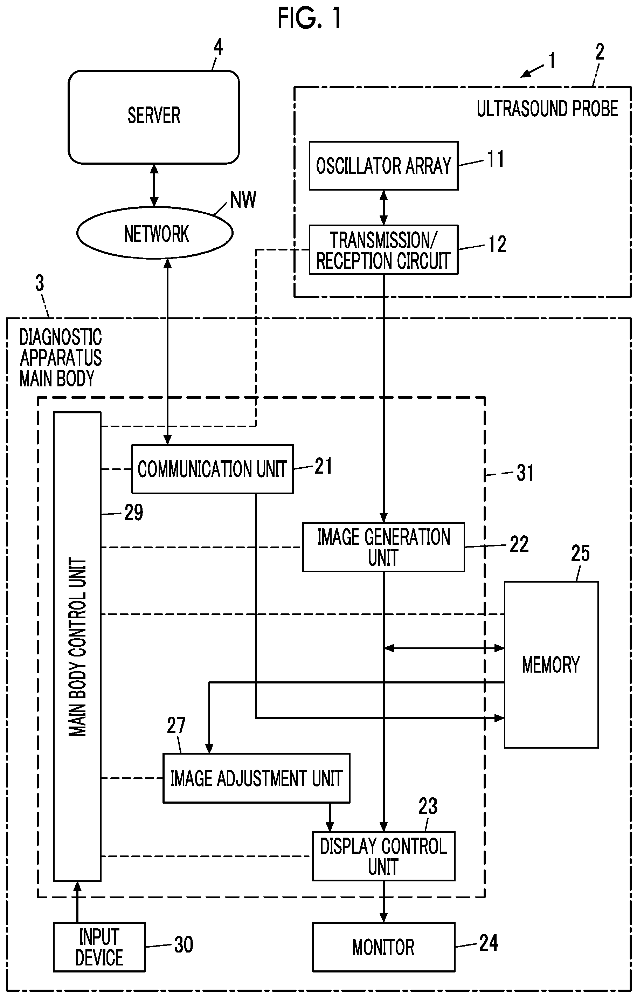

Fig. 1 shows a configuration of an ultrasound diagnostic apparatus 1 according to Embodiment 1 of the present invention. The ultrasound diagnostic apparatus 1 includes an ultrasound probe 2 and a diagnostic apparatus main body 3. The ultrasound probe 2 and the diagnostic apparatus main body 3 are connected to each other. The diagnostic apparatus main body 3 is connected to an external server 4 via a network NW.

The ultrasound probe 2 includes an oscillator array 11, and a transmission/reception circuit 12 is sequentially connected to the oscillator array 11.

The diagnostic apparatus main body 3 includes an image generation unit 22, and the image generation unit 22 is connected to the transmission/reception circuit 12 of the ultrasound probe 2. A display control unit 23 and a monitor 24 are sequentially connected to the image generation unit 22. A memory 25 is connected to the image generation unit 22. The diagnostic apparatus main body 3 includes a communication unit 21, and the communication unit 21 is connected to the server 4 via the network NW. The memory 25 is connected to the communication unit 21. An image adjustment unit 27 is connected to the memory 25. The display control unit 23 is connected to the image adjustment unit 27.

A main body control unit 29 is connected to the transmission/reception circuit 12, the communication unit 21, the image generation unit 22, the display control unit 23, the memory 25, and the image adjustment unit 27 of the ultrasound probe 2. An input device 30 is connected to the main body control unit 29.

A processor 31 is configured by the communication unit 21, the image generation unit 22, the display control unit 23, the image adjustment unit 27, and the main body control unit 29.

The server 4 is installed in, for example, a hospital, and is installed at a remote location with respect to a place where the diagnostic apparatus main body 3 is disposed. The server 4 manages image data and may be used in, for example, a so-called picture archiving and communication system (PACS).

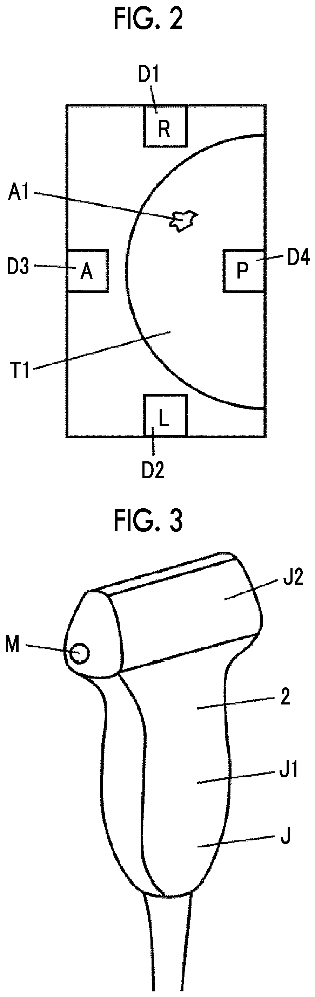

A radiation image T1 as shown in Fig. 2 captured by a radiation diagnostic apparatus (not shown) is stored in the server 4 in advance. The radiation image T1 stored in the server 4 includes a region of interest A1 suspected to be a lesion part. The radiation image T1 has a tag for storing information regarding the radiation image T1. This tag stores, for example, radiation image orientation information that is information regarding an orientation of a subject in the radiation image T1, such as a so-called anterior (A) direction, a posterior (P) direction, a right (R) direction, a left (L) direction, a head (H) direction, and a foot (F) direction.

As the tag of the radiation image T1, a so-called Digital Imaging and COmmunications in Medicine (DICOM) standard tag may be used.

In the example shown in Fig. 2, the radiation image T1 in which a breast of the subject is imaged from a so-called cranio caudal (CC) direction is shown. Four subject orientation marks indicating an orientation of the subject such as an R direction mark D1 representing the R direction, an L direction mark D2 representing the L direction, an A direction mark D3 representing the A direction, and a P direction mark D4 representing the P direction are disposed on the radiation image T1.

As shown in Fig. 3, the ultrasound probe 2 has a housing J including various electric circuits and the like and made of a resin or the like. The housing J has a grip portion J1 for gripping the ultrasound probe 2 by a user performing an examination on a subject, and a distal end portion J2 in which the oscillator array 11 is located. One protruding marker M is formed in the vicinity of the distal end portion J2 on one side portion of the housing J. The user can ascertain an orientation of the ultrasound probe 2 depending on an orientation in which the marker M is formed. An orientation of the subject in the ultrasound image, such as the A direction, the P direction, the R direction, the L direction, the H direction, or the F direction, is set with the orientation of the marker M as a reference.

The oscillator array 11 of the ultrasound probe 2 shown in Fig. 1 has a plurality of ultrasound oscillators arranged one-dimensionally or two-dimensionally. Each of these ultrasound oscillators transmits ultrasound in accordance with a drive signal supplied from the transmission/reception circuit 12, receives an ultrasound echo from a subject, and outputs a signal based on the ultrasound echo. Each ultrasound oscillator is configured by forming electrodes at both ends of a piezoelectric body made of, for example, a piezoelectric ceramic typified by lead zirconate titanate (PZT), a polymer piezoelectric element typified by poly vinylidene di fluoride (PVDF), and a piezoelectric single crystal typified by lead magnesium niobate-lead titanate (PMN-PT).

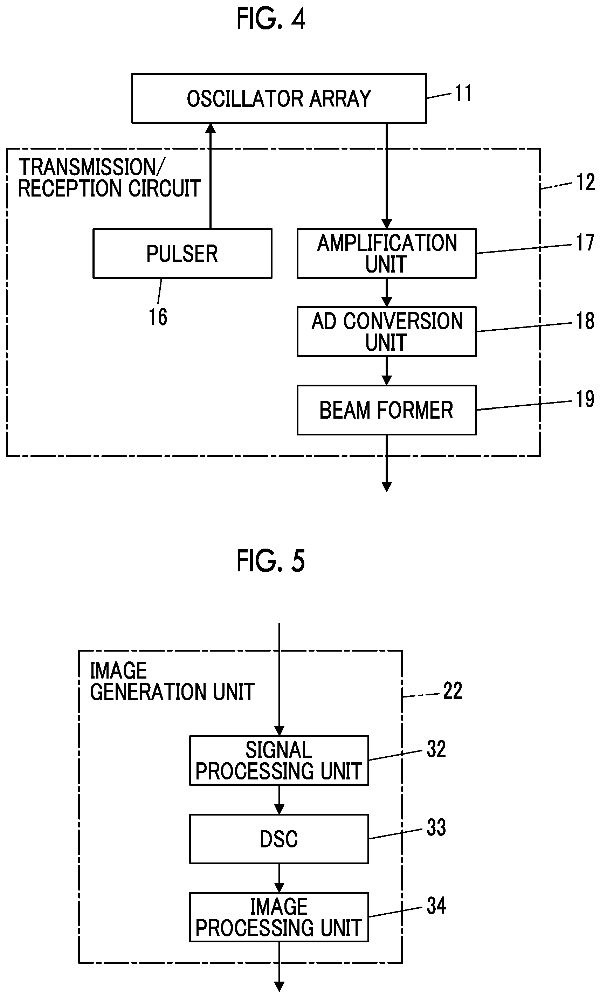

Under the control of the probe control unit 15, the transmission/reception circuit 12 transmits ultrasound from the oscillator array 11 and generates a sound ray signal on the basis of a received signal acquired by the oscillator array 11. As shown in Fig. 4, the transmission/reception circuit 12 includes a pulser 16 connected to the oscillator array 11, an amplification unit 17, an analog digital (AD) conversion unit 18, and a beam former 19 sequentially connected in series from the oscillator array 11.

The pulser 16 includes, for example, a plurality of pulse generators, and supplies respective drive signals of which delay amounts have been adjusted to the plurality of ultrasound oscillators such that ultrasound transmitted from the plurality of ultrasound oscillators of the oscillator array 11 forms an ultrasound beam on the basis of a transmission delay pattern selected in response to a control signal from the probe control unit 15. As described above, in a case where a pulsed or continuous wave voltage is applied to the electrodes of the ultrasound oscillators of the oscillator array 11, the piezoelectric body expands and contracts, and pulsed or continuous wave ultrasound is generated from the respective ultrasound oscillators, and an ultrasound beam is formed from combined waves of the ultrasound.

The transmitted ultrasound beam is reflected by, for example, a target such as a site of a subject and propagates toward the oscillator array 11 of the ultrasound probe 2. The ultrasound echo propagating toward the oscillator array 11 as described above is received by each of the ultrasound oscillators configuring the oscillator array 11. In this case, each of the ultrasound oscillators configuring the oscillator array 11 expands and contracts by receiving the propagating ultrasound echo to generate a received signal which is an electric signal, and these received signals are output to the amplification unit 17.

The amplification unit 17 amplifies the received signal input from each of the ultrasound oscillators configuring the oscillator array 11, and transmits the amplified received signal to the AD conversion unit 18. The AD conversion unit 18 converts the received signal transmitted from the amplification unit 17 into digital received data. The beam former 19 performs so-called reception focus processing by applying and adding a delay to each piece of the received data received from the AD conversion unit 18. Through this reception focus processing, each piece of the received data converted by the AD conversion unit 18 is subjected to phasing addition, and a sound ray signal in which a focus of the ultrasound echo is narrowed down is acquired.

The communication unit 21 is configured with a circuit including an antenna for transmitting and receiving radio waves, a circuit for performing a local area network (LAN) connection, and the like, and performs communication with the server 4 via the network NW under the control of the main body control unit 29. The communication unit 21 may receive the radiation image T1 and the like from the server 4 via the network NW.

As shown in Fig. 5, the image generation unit 22 has a configuration in which a signal processing unit 32, a digital scan converter (DSC) 33, and an image processing unit 34 are sequentially connected in series.

The signal processing unit 32 performs correction of attenuation based on a distance on the sound ray signal sent from the transmission/reception circuit 12 of the ultrasound probe 2 according to a depth of a reflection position of the ultrasound by using a sound velocity value set by the main body control unit 29, and then generates a B-mode image signal that is tomographic image information regarding a tissue in the subject by performing an envelope detection process.

The DSC 33 converts (raster conversion) the B-mode image signal generated by the signal processing unit 32 into an image signal according to a scanning method of a normal television signal.

The image processing unit 34 performs various types of necessary image processing such as gradation processing on the B-mode image signal input from the DSC 33, and then sends the B-mode image signal to the display control unit 23 and the memory 25. Hereinafter, the B-mode image signal that has undergone image processing by the image processing unit 34 will be referred to as an ultrasound image.

The memory 25 stores the ultrasound image generated by the image generation unit 22, the radiation image T1 transmitted from the server 4 to the communication unit 21 via the network NW, and the like. The ultrasound image stored in the memory 25 is read out under the control of the main body control unit 29 and sent to the display control unit 23 and the image adjustment unit 27. The radiation image T1 stored in the memory 25 is read out under the control of the main body control unit 29 and sent to the image adjustment unit 27.

As the memory 25, any of recording media such as a flash memory, a hard disk drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a Universal Serial Bus memory (USB memory) may be used.

The image adjustment unit 27 adjusts the radiation image T1 and the ultrasound image such that the region of interest A1 captured in the radiation image T1 and a region of interest captured in the ultrasound image are directed in an identical orientation on the basis of the radiation image orientation information stored in the tag of the radiation image T1 stored in the memory 25 and probe orientation information of the ultrasound probe 2 in a case where the ultrasound image is captured.

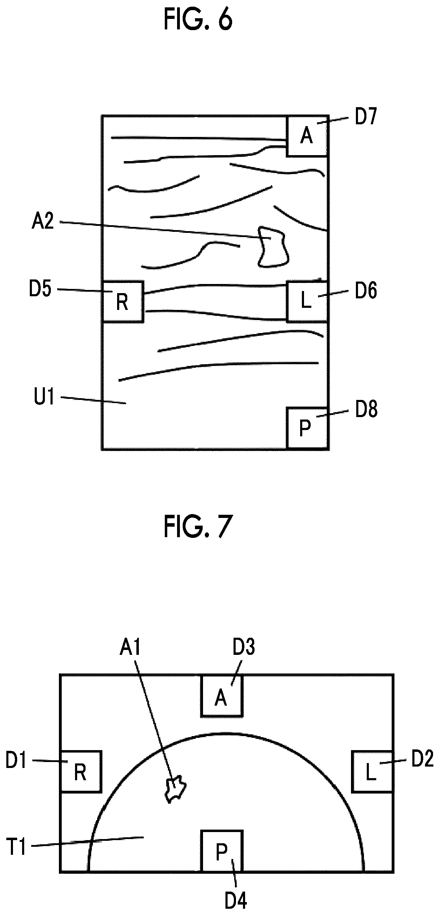

Here, the probe orientation information is information regarding an orientation of the subject in the ultrasound image, such as the A direction, the P direction, the R direction, the L direction, the H direction, and the F direction. The image adjustment unit 27 sets the probe orientation information on the basis of information input by an input operation of the user via the input device 30. Consequently, for example, as shown in Fig. 6, four subject orientation marks representing an orientation of the subject, such as an R direction mark D5 representing the R direction, an L direction mark D6 representing the L direction, an A direction mark D7 representing the A direction, and a P direction mark D8 representing the P direction are added to an ultrasound image U1. The ultrasound image U1 shown in Fig. 6 is an image of a breast of the subject.

The image adjustment unit 27 adjusts the R direction mark D1 and the R direction mark D5, the L direction mark D2 and the L direction mark D6, the A direction marks D3 and the A direction mark D7, and the P direction marks D4 and the P direction mark D8 to face an identical direction with respect to, for example, the radiation image T1 shown in Fig. 2 and the ultrasound image U1 shown in Fig. 6.

In this case, for example, the image adjustment unit 27 rotates the radiation image T1 shown in Fig. 2 by 90 degrees in a clockwise direction, that is, tilts the R direction mark D1 side toward the P direction mark D4 side, and then inverts the radiation image T1 left and right, that is, inverts the R direction mark D1 side and the L direction mark D2 side such that the radiation image T1 can be adjusted in the orientation shown in Fig. 7.

In the ultrasound image U1 shown in Fig. 6 and the radiation image T1 shown in Fig. 7, the R direction mark D1 and the R direction mark D5, the L direction mark D2 and the L direction mark D6, the A direction mark D3 and the A direction mark D7, and the P direction marks D4 and the P direction marks D8 each face an identical direction, and the region of interest A1 in the radiation image T1 and the region of interest A2 in the ultrasound image U1 also have an identical orientation.

The main body control unit 29 controls each unit of the diagnostic apparatus main body 3 according to a program or the like recorded in advance.

Under the control of the main body control unit 29, the display control unit 23 performs predetermined processing on the ultrasound image U1 generated by the image generation unit 22 and the radiation image T1 transmitted from the server 4 to the communication unit 21 via the network NW and displays the ultrasound image U1 and the radiation image T1 on the monitor 24.

The monitor 24 performs various types of display under the control of the display control unit 23. The monitor 24 includes, for example, a display device such as a liquid crystal display (LCD) or an organic electroluminescence display (organic EL display).

The input device 30 of the diagnostic apparatus main body 3 is used for the user to perform an input operation. The input device 30 is configured with, for example, a device such as a keyboard, a mouse, a track ball, a touch pad, and a touch panel used for the user to perform an input operation.

The processor 31 including the communication unit 21, the image generation unit 22, the display control unit 23, the image adjustment unit 27, and the main body control unit 29 is configured with a central processing unit (CPU) and a control program causing the CPU to perform various processes, but may be configured by using a field programmable gate array (FPGA), a digital signal processor (DSP), an application specific integrated circuit (ASIC), a graphics processing unit (GPU), or other integrated circuits (ICs), or may be configured by using a combination thereof.

The communication unit 21, the image generation unit 22, the display control unit 23, the image adjustment unit 27, and the main body control unit 29 may be partially or wholly integrated into one CPU or the like.

Hereinafter, an operation of the ultrasound diagnostic apparatus 1 according to the embodiment of the present invention will be described.

First, in step S1, the radiation image T1 stored in the server 4 is transmitted to the communication unit 21 via the network NW on the basis of an input operation or the like of the user via the input device 30, and the radiation image T1 is stored in the memory 25. As shown in Fig. 2, the radiation image T1 includes the region of interest A1 suspected to be a lesion part. The tag of the radiation image T1 stores the radiation image orientation information, and the R direction mark D1, the L direction mark D2, the A direction mark D3, and P direction mark D4 are disposed in the radiation image T1 on the basis of the radiation image orientation information.

Next, in step S2, the user captures a plurality of frames of the ultrasound image U in a state in which the ultrasound probe 2 comes into contact with a body surface of the subject.

In this case, the transmission/reception circuit 12 performs reception focus processing by using a preset sound velocity value under the control of the probe control unit 15 to generate a sound ray signal. The sound ray signal generated by the transmission/reception circuit 12 as described above is sent to the image generation unit 22. The image generation unit 22 generates the ultrasound image U1 as shown in Fig. 6 by using the sound ray signal sent from the transmission/reception circuit 12. The generated ultrasound image U1 is stored in the memory 25.

In the subsequent step S3, the image adjustment unit 27 sets probe orientation information on the basis of an input operation of the user via the input device 30. In this case, for example, the user inputs information regarding an orientation of the subject in the ultrasound image U1 while checking the orientation of the marker M provided on the ultrasound probe 2 as shown in Fig. 3 and the orientation of the subject. Consequently, for example, as shown in Fig. 6, the R direction mark D5, the L direction mark D6, the A direction mark D7, and the P direction mark D8 are disposed in the ultrasound image U1.

In step S4, the image adjustment unit 27 adjusts the radiation image T1 and the ultrasound image U1 such that the region of interest A1 captured in the radiation image T1 and a region of interest A2 captured in the ultrasound image U1 are directed in an identical orientation on the basis of the radiation image orientation information stored in the tag of the radiation image T1 stored in step S1 and the probe orientation information set in step S3.

The image adjustment unit 27 adjusts the radiation image T1 shown in Fig. 2 to the orientation shown in Fig. 7 by, for example, rotating the radiation image T1 clockwise and then inverting the radiation image T1 left and right. Consequently, the orientation of the region of interest A1 in the radiation image T1 shown in Fig. 7 and the orientation of the region of interest A2 in the ultrasound image U1 shown in Fig. 6 can be adjusted to be an identical orientation.

Consequently, it becomes easier for the user to compare the region of interest A1 in the radiation image T1 with the region of interest A2 in the ultrasound image U1.

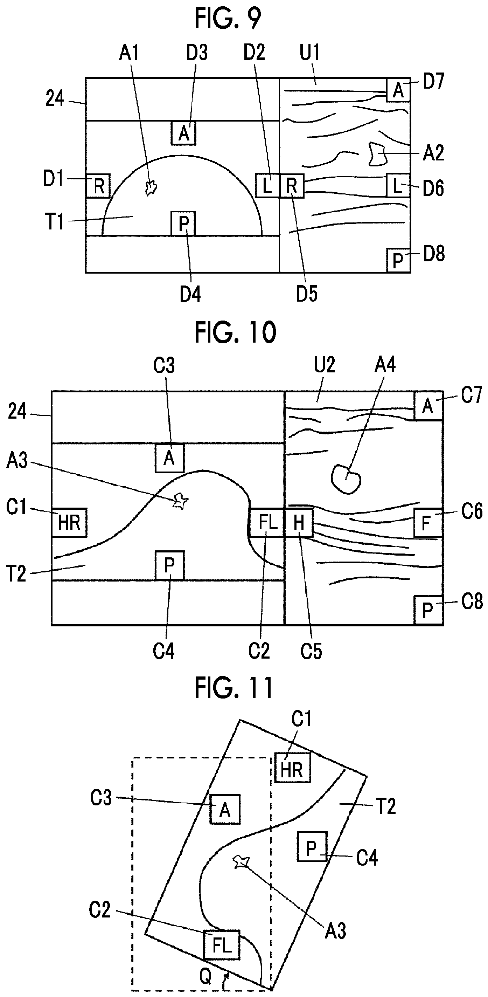

Finally, in step S5, as shown in Fig. 9, the radiation image T1 and the ultrasound image U1 adjusted in step S4 are displayed on the monitor 24.

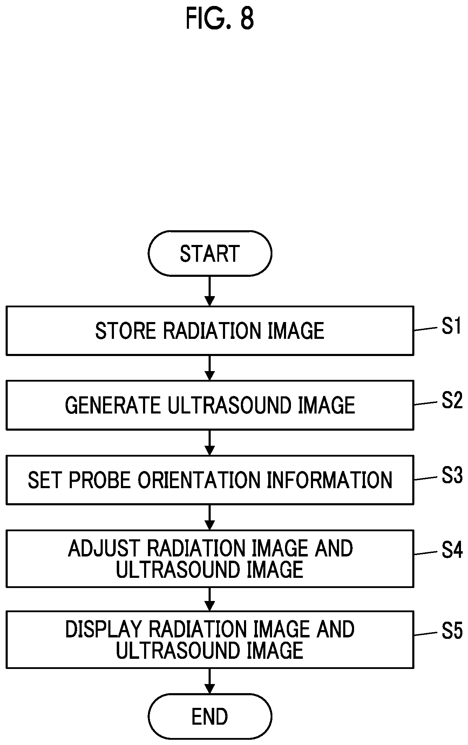

As described above, the operation of the ultrasound diagnostic apparatus 1 related to Embodiment 1 shown in the flowchart of Fig. 8 is completed.

As described above, according to the ultrasound diagnostic apparatus 1 related to Embodiment 1 of the present invention, the radiation image T1 and the ultrasound image U1 are adjusted such that the region of interest A1 in the radiation image T1 and the region of interest A2 in the ultrasound image U1 are directed in an identical orientation on the basis of the radiation image orientation information stored in the tag of the radiation image T1 and the probe orientation information of the ultrasound probe 2 in a case where the ultrasound image U1 is captured. Therefore, the user can easily compare the region of interest A1 in the radiation image T1 with the region of interest A2 in the ultrasound image U1, and can thus improve the diagnostic accuracy for the regions of interest A1 and A2.

In the ultrasound diagnostic apparatus 1, the image generation unit 22 is provided in the diagnostic apparatus main body 3, but may be provided in the ultrasound probe 2 instead of being provided in the diagnostic apparatus main body 3.

Although it has been described that the ultrasound probe 2 and the diagnostic apparatus main body 3 are connected to each other by wired communication, the ultrasound probe 2 and the diagnostic apparatus main body 3 may also be connected to each other by wireless communication.

Although the diagnostic apparatus main body 3 includes the single memory 25, a plurality of memories may be provided depending on an application or the like.

Although it has been described that the radiation image T1 is transmitted from the server 4 to the diagnostic apparatus main body 3 via the network NW, the radiation image T1 is not limited to being transmitted from the server 4. For example, the radiation image T1 may also be transmitted from a radiation diagnostic apparatus (not shown) to the diagnostic apparatus main body 3.

Although the protruding marker M is shown in Fig. 3, a shape of the marker M is not particularly limited as long as an orientation of the ultrasound probe 2 can be indicated. The marker M may have, for example, a recessed shape or may have a planar shape and a pattern.

It has been described that the R direction mark D1, the L direction mark D2, the A direction mark D3, the P direction mark D4, and the like are disposed in the radiation image T1, but a form of the mark representing a direction is not particularly limited to this. For example, by disposing a so-called schema for schematically representing a breast on the radiation image T1 and disposing a mark representing an orientation of a radiation source in a case where the radiation image T1 is captured on the schema, a direction in the radiation image T1 can be indicated.

For the ultrasound image U1, a form of a mark representing a direction is not limited to the R direction mark D5, the L direction mark D6, the A direction mark D7, the P direction mark D8, and the like. For example, by disposing a schema on the ultrasound image U1 and disposing a so-called probe mark representing a position and an orientation of the ultrasound probe 2 in a case where the ultrasound image U1 is captured on the schema, a direction in the ultrasound image U1 can be indicated.

A position and an orientation of the probe mark superimposed on the schema on the ultrasound image U1 may be set by an input operation of the user via the input device 30. In this case, the image adjustment unit 27 can set probe orientation information on the basis of an orientation of the probe mark input to the user.

The marks representing the directions in the radiation image T1 and the marks representing the directions in the ultrasound image U1 need not be displayed on the monitor 24. However, by displaying these marks on the monitor 24, the user can easily ascertain that an orientation of the region of interest A1 in the radiation image T1 and an orientation of the region of interest A2 in the ultrasound image U1 are identical.

Although an example in which the image adjustment unit 27 inverts the radiation image T1 left and right has been described, the inversion process is not particularly limited to left-right inversion. For example, the image adjustment unit 27 may also perform so-called upside-down processing of inverting the R-direction mark D1 side and the L-direction mark D2 side of the radiation image T1 shown in Fig. 2. As the inversion process, the image adjustment unit 27 is not limited to perform a process of inverting the radiation image T1 with respect to an axis that equally divides the radiation image T1 in the left-right direction or an axis that equally divides the radiation image T1 in the up-down direction, and may perform a process of setting any axis and inverting the radiation image T1 with respect to the axis.

In order to make an orientation of the region of interest A1 in the radiation image T1 and an orientation of the region of interest A2 in the ultrasound image U1 identical, an example of performing the rotation process and the inversion process on the radiation image T1 has been described. The ultrasound image U1 may be subjected to the rotation process and the inversion process instead of the radiation image T1, or both the radiation image T1 and the ultrasound image U1 may be subjected to the rotation process and the inversion process.

An example of adjusting the radiation image T1 captured from the CC direction and the ultrasound image U1 captured from the corresponding orientation has been described, but directions of capturing the radiation image T1 and the ultrasound image U1 are not particularly limited. For example, as shown in Fig. 10, a radiation image T2 in which a breast of a subject is imaged from a so-called medio lateral oblique (MLO) direction and an ultrasound image U2 captured from a corresponding orientation are rotated and inverted, and thus an orientation of a region of interest A3 in the radiation image T2 and an orientation of a region of interest A4 in the ultrasound image U2 can be made identical.

In the example in Fig. 10, in the radiation image T2 and the ultrasound image U2, a head right (HR) direction mark C1 and an H direction mark C5, a foot left (FL) direction mark C2 and an F direction mark C6, an A direction mark C3 and an A direction mark C7, and a P-direction mark C4 and a P direction mark C8 each face an identical direction.

For example, in a case of imaging a breast of a subject from the MLO direction, imaging of the breast is performed in a state in which a radiation source is rotated at any rotation angle. Therefore, in a case where the radiation image T2 is acquired by performing imaging from the MLO direction in which the radiation source is rotated at a rotation angle Q, as shown in Fig. 11, the image adjustment unit 27 can adjust the radiation image T2 and the ultrasound image U2 after performing rotational conversion on the radiation image T1 on the basis of the rotation angle Q of the radiation source.

The image adjustment unit 27 can adjust the radiation image T2 and the ultrasound image U2 not only in a case where the breast of the subject is imaged from the MLO direction but also in a case where the radiation image T1 is acquired in a state in which the radiation source is disposed in a direction inclined with respect to the vertical direction.

Consequently, the user can more easily compare the region of interest A3 in the radiation image T2 with the region of interest A4 in the ultrasound image U2, so that the diagnostic accuracy for the subject can be improved.

The tag of the radiation image T1 include radiation image breast information representing which of the left and right breasts is the breast of the subject captured in the radiation image T1, and for example, the user may input ultrasound image breast information representing which of the left and right breasts is the breast of the subject captured in the ultrasound image U1 via the input device 30.

In this state, the image adjustment unit 27 may adjust the radiation image T1 and the ultrasound image U1 such that an orientation of the region of interest A1 in the radiation image T1 and an orientation of the region of interest A2 in the ultrasound image U1 are identical only in a case where the breast of the subject captured in the radiation image T1 and the breast of the subject captured in the ultrasound image U1 match each other on the basis of the radiation image breast information and the ultrasound image breast information.

Consequently, the user can easily compare the region of interest A1 and the region of interest A2 in the radiation image T1 and the ultrasound image U1 in which an identical breast is captured, so that the diagnostic accuracy for the subject can be improved.

The image adjustment unit 27 may further adjust the radiation image T1 and the ultrasound image U1 that have already been adjusted and displayed on the monitor 24 on the basis of readjustment information input by the user via the input device 30. As a result, the radiation image T1 and the ultrasound image U1 can be readjusted such that the user can easily compare the images, and the diagnostic accuracy for the subject can be further improved.

Instead of rotating and inverting the entire radiation image T1 and the entire ultrasound image U1, the main body control unit 29 may rotate and invert a partial region including the region of interest A1 in the radiation image T1 and a partial region, designated by the user, including the region of interest A2 in the ultrasound image U1 and display the images on the monitor 24.

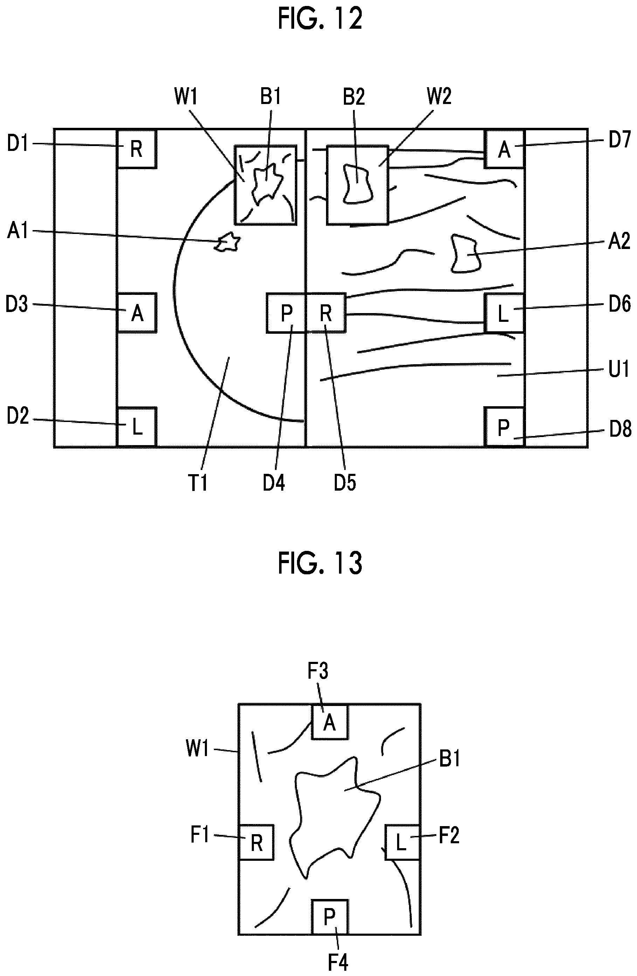

For example, as shown in Fig. 12, there may be a configuration in which a sub-window W1 is disposed on the radiation image T1, a sub-window W2 is disposed on the ultrasound image U1, a region of interest B1 in the radiation image T1 adjusted such that the orientation of the region of interest A1 and the orientation of the region of interest A2 are identical is displayed in the sub-window W1 on the radiation image T1, and a region of interest B2 in the ultrasound image U1 adjusted such that the orientation of the region of interest A1 and the orientation of the region of interest A2 are identical is displayed in the sub-window W2 on the ultrasound image U1.

Consequently, the user can easily compare the region of interest B1 with the region of interest B2 that face each other in an identical direction.

The main body control unit 29 enlarges and displays the region of interest B1 or the region of interest B2 such that the region of interest B1 in the sub-window W1 and the region of interest B2 in the sub-window W2 have an identical size on the basis of an inter-pixel distance of the radiation image T1 and an inter-pixel distance of the ultrasound image U1. Consequently, the user can more easily compare the region of interest B1 with the region of interest B2.

The main body control unit 29 enlarges both the region of interest B1 and the region of interest B2 such that the region of interest B1 in the sub-window W1 and the region of interest B2 in the sub-window W2 have an identical size.

Here, the inter-pixel distance of the radiation image T1 and the ultrasound image U1 is an actual length per pixel in each of the radiation image T1 and the ultrasound image U1. For example, in a case where the inter-pixel distance of the radiation image T1 is 0.1 mm and the inter-pixel distance of the ultrasound image U1 is 0.5 mm, a ratio of the size of the radiation image T1 to the size of the ultrasound image U1 may be determined as 0.1 mm/0.5 mm = 0.2.

Information regarding the inter-pixel distance of the ultrasound image U1 is stored in advance in, for example, the image adjustment unit 27. Information regarding the inter-pixel distance of the radiation image T1 is stored in advance in, for example, the tag of the radiation image T1.

For example, as shown in Fig. 13, the main body control unit 29 may display marks representing directions in the radiation image T1, such as an R direction mark F1, an L direction mark F2, an A direction mark F3, and a P direction mark F4 in the sub-window W1. As a result, the user can easily ascertain that adjustment such as rotation and inversion has been performed on the region of interest B1 in the sub-window W1.

For the same reason, the main body control unit 29 may display marks representing directions in the ultrasound image U1 in the sub-window W2 on the ultrasound image U1.

### Embodiment 2

In Embodiment 1, an example in which the region of interest A2 in the ultrasound image U1 is set on the basis of an input operation of a user via the input device 30 has been described. However, image analysis may be performed on the ultrasound image U1 such that the region of interest A2 is extracted.

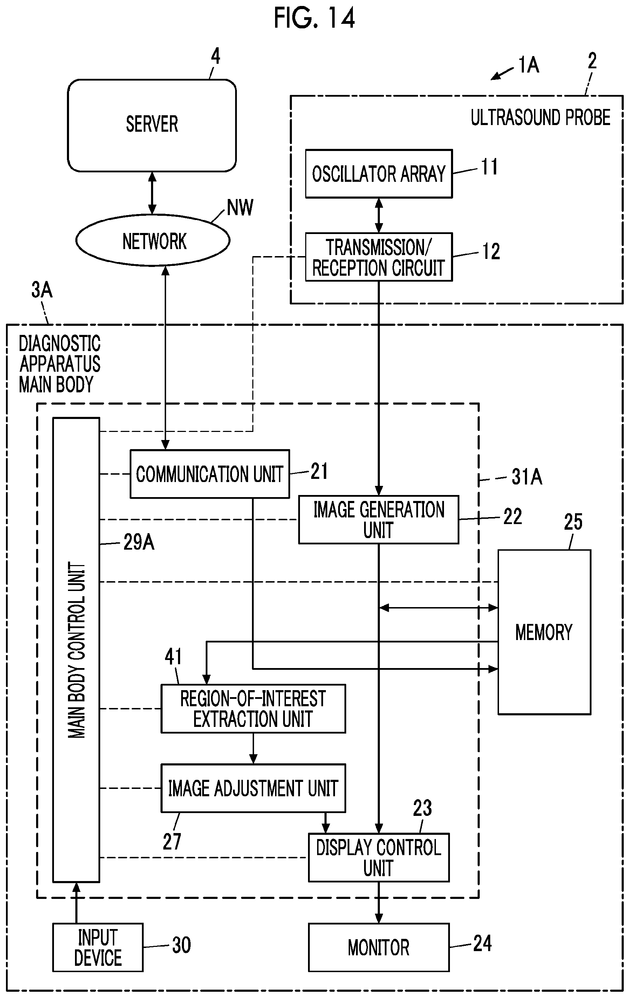

As shown in Fig. 14, an ultrasound diagnostic apparatus 1A according to Embodiment 2 includes a diagnostic apparatus main body 3A instead of the diagnostic apparatus main body 3 in the ultrasound diagnostic apparatus 1 according to Embodiment 1 shown in Fig. 1.

In the diagnostic apparatus main body 3A, a region-of-interest extraction unit 41 is added to the diagnostic apparatus main body 3 according to Embodiment 1, and a main body control unit 29A is provided instead of the main body control unit 29. Instead of the processor 31, a processor 31A including the region-of-interest extraction unit 41 is configured.

In the diagnostic apparatus main body 3A, the region-of-interest extraction unit 41 is connected to the memory 25. The image adjustment unit 27 and the main body control unit 29A are connected to the region-of-interest extraction unit 41.

The region-of-interest extraction unit 41 extracts the region of interest A2 in the ultrasound image U1 by performing image analysis on the ultrasound image U1 stored in the memory 25.

For example, the region-of-interest extraction unit 41 may store typical pattern data of the region of interest A2 as a template in advance, calculate a similarity for the pattern data while searching the ultrasound image U1, and consider that the region of interest A2 is present in a location where the similarity is equal to or more than a threshold value or the maximum.

As a method for extracting the region of interest A2, in addition to a method using simple template matching, for example, a machine learning method disclosed in Csurka et al.: Visual Categorization with Bags of Keypoints, Proc. of ECCV Workshop on Statistical Learning in Computer Vision, pp. 59 to 74 (2004), or a general image recognition method or the like using deep learning disclosed in Krizhevsk et al.: ImageNet Classification with Deep Convolutional Neural Networks, Advances in Neural Information Processing Systems 25, pp. 1106 to 1114 (2012) may be used.

The image adjustment unit 27 adjusts the radiation image T1 and the ultrasound image U1 such that the orientation of the region of interest A1 in the radiation image T1 and the orientation of the region of interest A2 in the ultrasound image U1 are identical.

From the above description, even in a case where the region of interest A2 in the ultrasound image U1 is extracted through image analysis, similarly to Embodiment 1, the radiation image T1 and the ultrasound image U1 are adjusted such that the orientation of the region of interest A1 in the radiation image T1 and the orientation of the region of interest A2 in the ultrasound image U1 are identical. Therefore, the user can easily compare the region of interest A1 in the radiation image T1 with the region of interest A2 in the ultrasound image U1, so that the diagnostic accuracy for the regions of interest A1 and A2 can be improved.

Instead of rotating and inverting the entire radiation image T1 and the entire ultrasound image U1, as shown in Fig. 12, the main body control unit 29A may rotate and invert a partial region including the region of interest A1 in the radiation image T1 and a partial region including the region of interest A2 extracted by the region-of-interest extraction unit 41 in the ultrasound image U1 and display the images on the monitor 24.

In this case, the main body control unit 29A may enlarge and display the region of interest B1 or the region of interest B2 such that the region of interest B1 in the sub-window W1 and the region of interest B2 in the sub-window W2 have an identical size on the basis of, for example, an inter-pixel distance of the radiation image T1 and an inter-pixel distance of the ultrasound image U1. Consequently, the user can more easily compare the region of interest B1 with the region of interest B2.

For example, as shown in Fig. 13, the main body control unit 29 may display marks representing directions in the radiation image T1, such as an R direction mark F1, an L direction mark F2, an A direction mark F3, and a P direction mark F4 in the sub-window W1. As a result, the user can easily ascertain that adjustment such as rotation and inversion has been performed on the region of interest B1 in the sub-window W1.

For the same reason, the main body control unit 29 may display marks representing directions in the ultrasound image U1 in the sub-window W2 on the ultrasound image U1.

The region-of-interest extraction unit 41 performs image analysis on the ultrasound image U1 to extract the region of interest A2 in the ultrasound image U1, but may also perform image analysis on the radiation image T1 to extract the region of interest A1 in the radiation image T1.

### Embodiment 3

In Embodiment 1, it has been described that the probe orientation information is set on the basis of an input operation of a user via the input device 30. However, for example, an orientation of the ultrasound probe 2 may be detected, and the probe orientation information may be set on the basis of the detected orientation of the ultrasound probe 2.

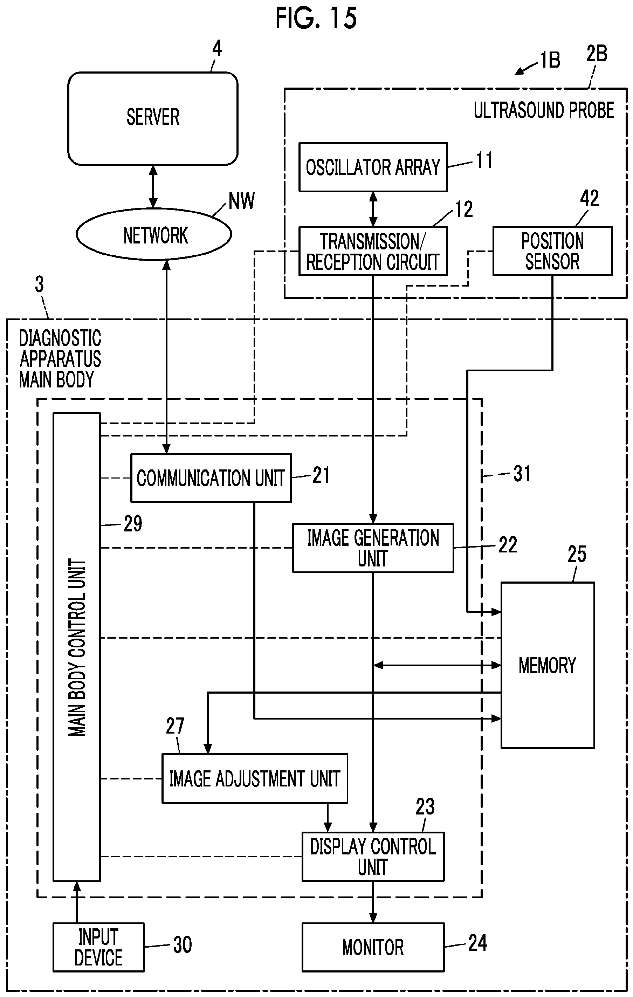

As shown in Fig. 15, an ultrasound diagnostic apparatus 1B according to Embodiment 3 includes an ultrasound probe 2B instead of the ultrasound probe 2 in the ultrasound diagnostic apparatus 1 according to Embodiment 1 shown in Fig. 1.

In the ultrasound probe 2B according to Embodiment 3, a position sensor 42 is added to the ultrasound probe 2 according to Embodiment 1. The position sensor 42 is connected to the memory 25 and the main body control unit 29 of the diagnostic apparatus main body 3.

The position sensor 42 is a sensor for detecting position information including an orientation of the ultrasound probe 2B, and may include, for example, a magnetic sensor, an optical position sensor, an acceleration sensor, a gyro sensor, or a global positioning system (GPS) sensor. The position information of the ultrasound probe 2B detected by the position sensor 42 is sent to the memory 25 of the diagnostic apparatus main body 3, and is stored in the memory 25 in association with the ultrasound image U1 each time the ultrasound image U1 is generated by the image generation unit 22 under the control of the main body control unit 29.

The image adjustment unit 27 receives the ultrasound image U1 and the radiation image T1 from the memory 25, sets probe orientation information on the basis of position information of the ultrasound probe 2B associated with the ultrasound image U1, and adjusts the radiation image T1 and the ultrasound image U1 such that an orientation of the region of interest A1 in the radiation image T1 and an orientation of the region of interest A2 in the ultrasound image U1 are identical on the basis of the probe orientation information and the radiation image orientation information of the radiation image T1.

From the above description, even in a case where the probe orientation information is set on the basis of the position information of the ultrasound probe 2B detected by the position sensor 42, similarly to Embodiment 1, the radiation image T1 and the ultrasound image U1 are adjusted such that the orientation of the region of interest A1 in the radiation image T1 and the orientation of the region of interest A2 in the ultrasound image U1 are identical. Therefore, the user can easily compare the region of interest A1 in the radiation image T1 with the region of interest A2 in the ultrasound image U1, so that the diagnostic accuracy for the regions of interest A1 and A2 can be improved.

### Explanation of References

1, 1A, 1B: ultrasound diagnostic apparatus
2, 2B: ultrasound probe
3, 3A: diagnostic apparatus main body
4: server
11: oscillator array
12: transmission/reception circuit
15: probe control unit
16: pulser
17: amplification unit
18: AD conversion unit
19: beam former
21: communication unit
22: image generation unit
23: display control unit
24: monitor
25: memory
27: image adjustment unit
29, 29A: main body control unit
30: input device
31, 31A: processor
32: signal processing unit
33: DSC
34: image processing unit
41: region-of-interest extraction unit
42: position sensor
A1 to A4, B1, B2: region of interest
C1: HR direction mark
C2: FL direction mark
C3, C7, D3, D7, F3: A direction mark
C4, C8, D4, D8, F4: P direction mark
C5: H direction mark
C6: F direction mark
D1, D5, F1: R direction mark
D2, D6, F2: L direction mark
J: housing
J1: grip portion
J2: distal end portion
M: marker
NW: network
Q: rotation angle
T1, T2: radiation image
U1, U2: ultrasound image
W1, W2: sub-window

## Claims

1. An ultrasound diagnostic apparatus (1) comprising:
an ultrasound probe (2);
an image generation unit (22) that generates an ultrasound image including a region of interest of a breast of a subject captured in a radiation image by transmitting and receiving ultrasound beams to and from the subject by using the ultrasound probe (2);
an image adjustment unit (27) that adjusts the radiation image and the ultrasound image such that the region of interest captured in the ultrasound image and the region of interest captured in the radiation image have an identical orientation on the basis of radiation image orientation information stored in a tag of the radiation image and probe orientation information of the ultrasound probe (2) in a case where the ultrasound image is captured, wherein the image adjustment unit (27) determines a ratio between sizes of the radiation image and ultrasound image on the basis of a stored inter-pixel distance of the radiation image and a stored inter-pixel distance of the ultrasound image, and generates the adjusted radiation image and ultrasound image such that the region of interest captured in the radiation image and the region of interest captured in the ultrasound image have an identical size; and
a monitor (24) that displays the radiation image and the ultrasound image that have been adjusted by the image adjustment unit (27).

2. The ultrasound diagnostic apparatus (1) according to claim 1,
wherein the probe orientation information is position information of the ultrasound probe (2) designated by a user or position information detected by a position sensor (42) mounted on the ultrasound probe (2).

3. The ultrasound diagnostic apparatus (1) according to claim 1 or 2,
wherein the image adjustment unit (27) generates the adjusted radiation image and ultrasound image by performing at least one of a rotation process or an inversion process on at least one of an entire radiation image or an entire ultrasound image.

4. The ultrasound diagnostic apparatus (1) according to claim 1 or 2, further comprising:
a region-of-interest extraction unit (41) that extracts the region of interest from each of the radiation image and the ultrasound image,
wherein the image adjustment unit (27) generates the adjusted radiation image and ultrasound image by performing at least one of a rotation process or an inversion process on at least one of the region of interest extracted from the radiation image or the region of interest extracted from the ultrasound image.

5. The ultrasound diagnostic apparatus (1) according to any one of claims 1 to 4,
wherein the image adjustment unit (27) displays a subject orientation mark representing an orientation of the subject on the adjusted radiation image and ultrasound image to be superimposed.

6. The ultrasound diagnostic apparatus (1) according to any one of claims 1 to 5,
wherein, in a case where the radiation image is acquired in a state in which a radiation source is disposed in a direction inclined with respect to a vertical direction, the image adjustment unit (27) performs rotational conversion on the radiation image on the basis of a rotation angle of the radiation source and then adjusts the radiation image and the ultrasound image.

7. The ultrasound diagnostic apparatus (1) according to any one of claims 1 to 6,
wherein the tag of the radiation image includes radiation image breast information indicating whether the breast of the subject captured in the radiation image is a left or right breast, and
in a case where the breast of the subject captured in the radiation image and the breast of the subject captured in the ultrasound image match each other, the image adjustment unit (27) adjusts the radiation image and the ultrasound image on the basis of the radiation image breast information and information input by a user and representing whether the breast of the subject captured in the ultrasound image is a left or right breast.

8. The ultrasound diagnostic apparatus (1) according to any one of claims 1 to 7,
wherein the image adjustment unit (27) further adjusts the already adjusted radiation image and ultrasound image on the basis of readjustment information input by a user.

9. A control method for an ultrasound diagnostic apparatus (1), the method comprising:
generating an ultrasound image including a region of interest of a breast of a subject captured in a radiation image by transmitting and receiving ultrasound beams to and from the subject by using an ultrasound probe (2);
adjusting the radiation image and the ultrasound image such that the region of interest captured in the ultrasound image and the region of interest captured in the radiation image have an identical orientation on the basis of radiation image orientation information stored in a tag of the radiation image and probe orientation information of the ultrasound probe (2) in a case where the ultrasound image is captured;
determining a ratio between sizes of the radiation image and ultrasound image on the basis of a stored inter-pixel distance of the radiation image and a stored inter-pixel distance of the ultrasound image, and generating the adjusted radiation image and ultrasound image such that the region of interest captured in the radiation image and the region of interest captured in the ultrasound image have an identical size; and
displaying the radiation image and the ultrasound image that have been adjusted on a monitor (24).

## Patentansprüche

1. Ultraschalldiagnosevorrichtung (1), umfassend:
eine Ultraschallsonde (2);
eine Bilderzeugungseinheit (22), die ein Ultraschallbild, das einen Bereich von Interesse einer Brust einer Untersuchungsperson, der in einem Strahlungsbild aufgenommen worden ist, enthält, erzeugt, indem sie Ultraschallstrahlen an die Untersuchungsperson überträgt und von dieser empfängt, indem sie die Ultraschallsonde (2) verwendet;
eine Bilderanpassungseinheit (27), die das Strahlungsbild und das Ultraschallbild so anpasst, dass der Bereich von Interesse, der in dem Ultraschallbild aufgenommen worden ist, und der Bereich von Interesse, der in dem Strahlungsbild aufgenommen worden ist, eine identische Ausrichtung auf der Grundlage von Strahlungsbild-Ausrichtungsinformationen, die in einem Etikett des Strahlungsbildes gespeichert sind,
und Sonden-Ausrichtungsinformationen der Ultraschallsonde (2) in einem Fall, in dem das Ultraschallbild aufgenommen wird, aufweisen, wobei die Bilderanpassungseinheit (27) ein Verhältnis zwischen Größen des Strahlungsbildes und Ultraschallbildes auf der Grundlage einer gespeicherten Inter-Pixel-Distanz des Strahlungsbildes und einer gespeicherten Inter-Pixel-Distanz des Ultraschallbildes bestimmt und das angepasste Strahlungsbild und Ultraschallbild so erzeugt, dass der Bereich von Interesse, der in dem Strahlungsbild aufgenommen worden ist, und der Bereich von Interesse, der in dem Ultraschallbild aufgenommen worden ist, eine identische Größe aufweisen; und einen Monitor (24), der das Strahlungsbild und das Ultraschallbild, die von der Bilderanpassungseinheit (27) angepasst worden sind, anzeigt.

2. Ultraschalldiagnosevorrichtung (1) nach Anspruch 1,
wobei die Sonden-Ausrichtungsinformationen Positionsinformationen der Ultraschallsonde (2), die von einem Benutzer designiert sind, oder Positionsinformationen, die von einem Positionssensor (42), der an der Ultraschallsonde (2) montiert ist, detektiert werden, sind.

3. Ultraschalldiagnosevorrichtung (1) nach Anspruch 1 oder 2,
wobei die Bilderanpassungseinheit (27) das angepasste Strahlungsbild und Ultraschallbild erzeugt, indem sie mindestens eines von einem Drehprozess und einem Inversionsprozess an mindestens einem von einem gesamten Strahlungsbild und einem gesamten Ultraschallbild durchführt.

4. Ultraschalldiagnosevorrichtung (1) nach Anspruch 1 oder 2, ferner umfassend:
eine Bereich-von-Interesse-Auswerteeinheit (41), die den Bereich von Interesse aus jeweils dem Strahlungsbild und dem Ultraschallbild extrahiert,
wobei die Bilderanpassungseinheit (27) das angepasste Strahlungsbild und
Ultraschallbild erzeugt, in dem sie mindestens eines von einem Drehprozess und einem Inversionsprozess an mindestens einem von dem Bereich von Interesse, der aus dem Strahlungsbild extrahiert worden ist, und dem Bereich von Interesse, der aus dem Ultraschallbild extrahiert worden ist, durchführt.

5. Ultraschalldiagnosevorrichtung (1) nach einem der Ansprüche 1 bis 4,
wobei die Bilderanpassungseinheit (27) eine Subjektausrichtungsmarkierung, die eine Ausrichtung der Untersuchungsperson darstellt, auf dem angepassten Strahlungsbild und Ultraschallbild, die überlagert werden sollen, anzeigt.

6. Ultraschalldiagnosevorrichtung (1) nach einem der Ansprüche 1 bis 5,
wobei in einem Fall, in dem das Strahlungsbild in einem Zustand erfasst wird, in dem eine Strahlungsquelle in einer Richtung, die in Bezug auf eine vertikale Richtung geneigt ist, angeordnet ist, die Bilderanpassungseinheit (27) Drehung des Strahlungsbildes auf der Grundlage eines Drehwinkels der Strahlungsquelle durchführt und dann das Strahlungsbild und das Ultraschallbild anpasst.

7. Ultraschalldiagnosevorrichtung (1) nach einem der Ansprüche 1 bis 6,
wobei das Etikett des Strahlungsbildes Strahlungsbild-Brustinformationen enthält, die angeben, ob die in dem Strahlungsbild aufgenommene Brust der Untersuchungsperson eine linke oder rechte Brust ist, und
in einem Fall, in dem die Brust der Untersuchungsperson, die in dem Strahlungsbild aufgenommen worden ist, und die Brust der Untersuchungsperson, die in dem Ultraschallbild aufgenommen worden ist, miteinander übereinstimmen, die Bilderanpassungseinheit (27) das Strahlungsbild und das Ultraschallbild auf der Grundlage der Strahlungsbild-Brustinformationen und von einem Benutzer eingegebener Informationen, die angeben, ob die in dem Ultraschallbild aufgenommene Brust der Untersuchungsperson eine linke oder rechte Brust ist, anpasst.

8. Ultraschalldiagnosevorrichtung (1) nach einem der Ansprüche 1 bis 7,
wobei die Bilderanpassungseinheit (27) das bereits angepasste Strahlungsbild und Ultraschallbild auf der Grundlage von von einem Benutzer eingegebenen Wiederanpassungsinformationen weiter anpasst.

9. Steuerverfahren einer Ultraschalldiagnosevorrichtung, (1) wobei das Verfahren umfasst:
Erzeugen eines Ultraschallbildes, das einen Bereich von Interesse einer Brust einer Untersuchungsperson, die in einem Strahlungsbild aufgenommen worden ist, enthält, durch Senden und Empfangen von Ultraschallstrahlen an die Untersuchungsperson und von dieser durch Verwenden einer Ultraschallsonde (2);
Anpassen des Strahlungsbildes und des Ultraschallbildes so, dass der Bereich von Interesse, der in dem Ultraschallbild aufgenommen worden ist, und der Bereich von Interesse, der in dem Strahlungsbild aufgenommen worden ist, eine identische Ausrichtung auf der Grundlage von Strahlungsbild-Ausrichtungsinformationen, die in einem Etikett des Strahlungsbildes gespeichert sind, und von Ultraschallsonden-Ausrichtungsinformationen der Ultraschallsonde (2) in einem Fall, in dem das Ultraschallbild aufgenommen wird, aufweisen;
Bestimmen eines Verhältnisses zwischen Größen des Strahlungsbildes und Ultraschallbildes auf der Grundlage einer gespeicherten Abstand zwischen Pixeln des Strahlungsbildes und eines gespeicherten Abstand zwischen Pixeln des Ultraschallbildes und Erzeugen des angepassten Strahlungsbildes und Ultraschallbildes so, dass der Bereich von Interesse, der in dem Strahlungsbild aufgenommen worden ist, und der Bereich von Interesse, der in dem Ultraschallbild aufgenommen worden ist, eine identische Größe aufweisen; und
Anzeigen des Strahlungsbildes und des Ultraschallbildes, die angepasst worden sind, auf einem Monitor (24).

## Revendications

1. Appareil de diagnostic par ultrasons (1) comprenant :
une sonde ultrasonore (2) ;
une unité de génération d'images (22) qui génère une image ultrasonore incluant une région d'intérêt d'un sein d'un sujet capturée dans une image de rayonnement en transmettant et en recevant des faisceaux ultrasonores vers et depuis le sujet en utilisant la sonde ultrasonore (2) ;
une unité d'ajustement d'images (27) qui ajuste l'image de rayonnement et l'image ultrasonore de sorte que la région d'intérêt capturée dans l'image ultrasonore et la région d'intérêt capturée dans l'image de rayonnement présentent une orientation identique sur la base d'informations d'orientation d'image de rayonnement stockées dans une étiquette de l'image de rayonnement et d'informations d'orientation de sonde de la sonde ultrasonore (2) dans un cas où l'image ultrasonore est capturée, où l'unité d'ajustement d'images (27) détermine un rapport entre des tailles de l'image de rayonnement et de l'image ultrasonore sur la base d'une distance inter-pixel stockée de l'image de rayonnement et d'une distance inter-pixel stockée de l'image ultrasonore, et génère l'image de rayonnement et l'image ultrasonore ajustées de sorte que la région d'intérêt capturée dans l'image de rayonnement et la région d'intérêt capturée dans l'image ultrasonore présentent une taille identique ; et
un moniteur (24) qui affiche l'image de rayonnement et l'image ultrasonore qui ont été ajustées par l'unité d'ajustement d'images (27).

2. Appareil de diagnostic par ultrasons (1) selon la revendication 1,
dans lequel les informations d'orientation de sonde sont des informations de position de la sonde ultrasonore (2) désignées par un utilisateur ou des informations de position détectées par un capteur de position (42) monté sur la sonde ultrasonore (2).

3. Appareil de diagnostic par ultrasons (1) selon la revendication 1 ou la revendication 2, dans lequel l'unité d'ajustement d'images (27) génère l'image de rayonnement et l'image ultrasonore ajustées en effectuant au moins l'un d'un processus de rotation et d'un processus d'inversion sur au moins l'une d'une image de rayonnement entière et d'une image ultrasonore entière.

4. Appareil de diagnostic par ultrasons (1) selon la revendication 1 ou la revendication 2, comprenant en outre :
une unité d'extraction de région d'intérêt (41) qui extrait la région d'intérêt de chacune de l'image de rayonnement et de l'image ultrasonore,
dans lequel l'unité d'ajustement d'images (27) génère l'image de rayonnement ajustée et l'image ultrasonore ajustée en effectuant au moins l'un d'un processus de rotation et d'un processus d'inversion sur au moins l'une de la région d'intérêt extraite de l'image de rayonnement et de la région d'intérêt extraite de l'image ultrasonore.

5. Appareil de diagnostic par ultrasons (1) selon l'une quelconque des revendications 1 à 4,
dans lequel l'unité d'ajustement d'images (27) affiche un repère d'orientation de sujet représentant une orientation du sujet sur l'image de rayonnement ajustée et l'image ultrasonore ajustée à superposer.

6. Appareil de diagnostic par ultrasons (1) selon l'une quelconque des revendications 1 à 5,
dans lequel, dans un cas où l'image de rayonnement est acquise dans un état où une source de rayonnement est disposée dans une direction inclinée par rapport à une direction verticale, l'unité d'ajustement d'images (27) effectue une conversion rotationnelle sur l'image de rayonnement sur la base d'un angle de rotation de la source de rayonnement, puis ajuste l'image de rayonnement et l'image ultrasonore.

7. Appareil de diagnostic par ultrasons (1) selon l'une quelconque des revendications 1 à 6,
dans lequel l'étiquette de l'image de rayonnement inclut des informations de sein d'image de rayonnement indiquant si le sein du sujet capturé dans l'image de rayonnement est un sein gauche ou un sein droit, et
dans un cas où le sein du sujet capturé dans l'image de rayonnement et le sein du sujet capturé dans l'image ultrasonore correspondent l'un à l'autre, l'unité d'ajustement d'images (27) ajuste l'image de rayonnement et l'image ultrasonore sur la base des informations de sein d'image de rayonnement et des informations entrées par un utilisateur et représentant si le sein du sujet capturé dans l'image ultrasonore est un sein gauche ou un sein droit.

8. Appareil de diagnostic par ultrasons (1) selon l'une quelconque des revendications 1 à 7,
dans lequel l'unité d'ajustement d'images (27) ajuste en outre l'image de rayonnement et l'image ultrasonore déjà ajustées sur la base d'informations de réajustement entrées par un utilisateur.

9. Procédé de contrôle pour un appareil de diagnostic par ultrasons (1), le procédé comprenant :
générer une image ultrasonore incluant une région d'intérêt d'un sein d'un sujet capturée dans une image de rayonnement en transmettant et en recevant des faisceaux ultrasonores vers et depuis le sujet en utilisant une sonde ultrasonore (2) ;
ajuster l'image de rayonnement et l'image ultrasonore de sorte que la région d'intérêt capturée dans l'image ultrasonore et la région d'intérêt capturée dans l'image de rayonnement présentent une orientation identique sur la base d'informations d'orientation d'image de rayonnement stockées dans une étiquette de l'image de rayonnement et d'informations d'orientation de sonde de la sonde ultrasonore (2) dans un cas où l'image ultrasonore est capturée ;
déterminer un rapport entre des tailles de l'image de rayonnement et de l'image ultrasonore sur la base d'une distance inter-pixel stockée de l'image de rayonnement et d'une distance inter-pixel stockée de l'image ultrasonore, et générer l'image de rayonnement et l'image ultrasonore ajustées de sorte que la région d'intérêt capturée dans l'image de rayonnement et la région d'intérêt capturée dans l'image ultrasonore présentent une taille identique ; et
afficher l'image de rayonnement et l'image ultrasonore qui ont été ajustées sur un moniteur (24).
